# EUROPEAN PATENT APPLICATION

(11) **EP 1 400 596 A1**
(43) Date of publication of application: **24.03.2004**
(21) Application number: 03016841.3
(22) Date of filing: 24.07.2003
(51) Int. Cl.: C12Q 1/68, C07H 21/00

(54) **Androgen receptor complex-associated protein**

(30) Priority: 25.07.2002 US 205737
(71) Applicant: Taipei-Veterans General Hospital, Taipei (TW); TargetGen, Inc., Taipei 110 (TW)
(72) Inventor: Chang, Tai-Jay, Taipei (TW)
(74) Representative: Brandenburg, Thomas, Dr.

(57) **Abstract**

Primers and probes for detecting the expression of the human ARCAP gene.

SEQUENCE LISTING

<110> Chang, Tai-Jay

<120> ANDROGEN RECEPTOR COMPLEX-ASSOCIATED
      PROTEIN

<130> 14321-002001

<140> US 10/205,737
<141> 2002-07-25

<150> US 09/781,693
<151> 2001-02-12

<150> US 60/262,312
<151> 2001-01-17

<160> 22

<170> FastSEQ for Windows Version 4.0

<210> 1
<211> 3016
<212> DNA
<213> Homo sapiens

<400> 1
ccggctcagg cagagccatg tctcggggtg gctcctaccc acacctgttg tgggacgtga 60
ggaaaaggtc cctcgggctg gaggacccgt cccggctgcg gagtcgctac ctgggaagaa 120
gagaatttat ccaaagatta aaacttgaag caacccttaa tgtgcatgat ggttgtgtta 180
atacaatctg ttggaatgac actggagaat atattttatc tggctcagat gacaccaaat 240
tagtaattag taatccttac agcagaaagg ttttgacaac aattcgttca gggcaccgag 300
caaacatatt tagtgcaaag ttcttacctt gtacaaatga taaacagatt gtatcctgct 360
ctggagatgg agtaatattt tataccaacg ttgagcaaga tgcagaaacc aacagacaat 420
gccaatttac gtgtcattat ggaactactt atgagattat gactgtaccc aatgaccctt 480
acacttttct ctcttgtggt gaagatggaa ctgttaggtg gtttgataca cgcatcaaaa 540
ctagctgcac aaaagaagat tgtaaagatg atattttaat taactgtcga cgtgctgcca 600
cgtctgttgc tatttgccca ccaataccat attaccttgc tgttggttgt tctgacagct 660
cagtacgaat atatgatcgg cgaatgctgg gcacaagagc tacagggaat tatgcaggtc 720
gagggactac tggaatggtt gcccgtttta ttccttccca tcttaataat aagtcctgca 780
gagtgacatc tctgtgttac agtgaagatg gtcaagagat tctcgttagt tactcttcag 840
attacatata tctttttgac ccgaaagatg atacagcacg agaacttaaa actccttctg 900
cggaagagag aagagaagag ttgcgacaac caccagttaa gcgtttgaga cttcgtggtg 960
attggtcaga tactggaccc agagcaaggc cggagagtga acgagaacga gatggagagc 1020
agagtcccaa tgtgtcattg atgcagagaa tgtctgatat gttatcaaga tggtttgaag 1080
aagcaagtga ggttgcacaa agcaatagag gacgaggaag atctcgaccc agaggtggaa 1140
caagtcaatc agatatttca actcttccta cggtcccatc aagtcctgat ttggaagtga 1200
gtgaaactgc aatggaagta gatactccag ctgaacaatt tcttcagcct tctacatcct 1260
ctacaatgtc agctcaggct cattcgacat catctcccac agaaagccct cattctactc 1320
ctttgctatc ttctccagac agtgaacaaa ggcagtctgt tgaggcatct ggacaccaca 1380
cacatcatca gtctgataac aataatgaaa agctgagccc caaaccaggg acaggtgaac 1440
cagttttaag tttgcactac agcacagaag gaacaactac aagcacaata aaactgaact 1500
ttacagatga atggagcagt atagcatcaa gttctagagg aattgggagc cattgcaaat 1560
ctgagggtca ggaggaatct ttcgtcccac agagctcagt gcaaccacca gaaggagaca 1620
gtgaaacaaa agctcctgaa gaatcatcag aggatgtgac aaaatatcag gaaggagtat 1680
ctgcagaaaa cccagttgag aaccatatca atataacaca atcagataag ttcacagcca 1740
agccattgga ttccaactca ggagaaagaa atgacctcaa tcttgatcgc tcttgtgggg 1800
ttccagaaga atctgcttca tctgaaaaag ccaaggaacc agaaacttca gatcagacta 1860
gcactgagag tgctaccaat gaaaataaca ccaatcctga gcctcagttc caaacagaag 1920
ccactgggcc ttcagctcat gaagaaacat ccaccaggga ctctgctctt caggacacag 1980
atgacagtga tgatgaccca gtcctgatcc caggtgcaag gtatcgagca ggacctggtg 2040
atagacgctc tgctgttgcc cgtattcagg agttcttcag acggagaaaa gaaaggaaag 2100
aaatggaaga attggatact ttgaacatta gaaggccgct agtaaaaatg gtttataaag 2160
gccatcgcaa ctccaggaca atgataaaag aagccaattt ctggggtgct aactttgtaa 2220
tgagtggttc tgactgtggc cacattttca tctgggatcg gcacactgct gagcatttga 2280

tgcttctgga agctgataat catgtggtaa actgcctgca gccacatccg tttgacccaa 2340
ttttagcctc atctggcata gattatgaca taaagatctg gtcaccatta gaagagtcaa 2400
ggatttttaa ccgaaaactt gctgatgaag ttataactcg aaacgaactc atgctggaag 2460
aaactagaaa caccattaca gttccagcct ctttcatgtt gaggatgttg gcttcactta 2520
atcatatccg agctgaccgg ttggagggtg acagatcaga aggctctggt caagagaatg 2580
aaaatgagga tgaggaataa taaactcttt ttggcaagca cttaaatgtt ctgaaatttg 2640
tataagacat ttattatatt tttttcttta cagagcttta gtgcaatttt aaggttatgg 2700
tttttggagt ttttcccttt ttttgggata acctaacatt ggtttggaat gattgtgtgc 2760
atgaatttgg gagattgtat aaaacaaaac tagcagaatg tttttaaaac tttttgccgt 2820
gtatgaggag tgctagaaaa tgcaaagtgc aatattttcc ctaaccttca aatgtgggag 2880
cttggatcaa tgttgaagaa taattttcat catagtgaaa atgttggttc aaataaattt 2940
ctacacttgc catttgcatg tttgttgctt tctaattaaa gaaactggtt gttttaaaaa 3000
aaaaaaaaag gaattc 3016

<210> 2
<211> 24
<212> DNA
<213> Artificial Sequence

<220>
<223> Primer

<400> 2
atgtctcggg gtggctccta ccca 24

<210> 3
<211> 26
<212> DNA
<213> Artificial Sequence

<220>
<223> Primer

<400> 3
atgactgtac ccaatgaccc ttacac 26

<210> 4
<211> 26
<212> DNA
<213> Artificial Sequence

<220>
<223> Primer

<400> 4
acagctcagt acgaatatat gatcgg 26

<210> 5
<211> 20
<212> DNA
<213> Artificial Sequence

<220>
<223> Primer

<400> 5
ctactggaat ggttgccgtt 20

<210> 6
<211> 26
<212> DNA
<213> Artificial Sequence

<220>
<223> Primer

<400> 6
atgcagagaa tgtctgatat gttatc 26

<210> 7
<211> 27
<212> DNA
<213> Artificial Sequence

<220>
<223> Primer

<400> 7
tccaactcag gagaaagaaa tgacctc                                          27

<210> 8
<211> 20
<212> DNA
<213> Artificial Sequence

<220>
<223> Primer

<400> 8
gccactgggc cttcagctca                                                  20

<210> 9
<211> 21
<212> DNA
<213> Artificial Sequence

<220>
<223> Primer

<400> 9
gtgctaactt tgtaatgagt g                                                21

<210> 10
<211> 26
<212> DNA
<213> Artificial Sequence

<220>
<223> Primer

<400> 10
acagttccat cttcaccaca agagag                                           26

<210> 11
<211> 26
<212> DNA
<213> Artificial Sequence

<220>
<223> Primer

<400> 11
ccgatcatat attcgtactg agctgt                                           26

<210> 12
<211> 27
<212> DNA
<213> Artificial Sequence

<220>
<223> Primer

<400> 12
gaccgtagga agagttgaaa tatctga                                          27

<210> 13

<211> 25
<212> DNA
<213> Artificial Sequence

<220>
<223> Primer

<400> 13
actggttcac ctgtccctgg tttgg 25

<210> 14
<211> 27
<212> DNA
<213> Artificial Sequence

<220>
<223> Primer

<400> 14
gaggtcattt ctttctcctg agttgga 27

<210> 15
<211> 20
<212> DNA
<213> Artificial Sequence

<220>
<223> Primer

<400> 15
tgagctgaag gcccagtggc 20

<210> 16
<211> 30
<212> DNA
<213> Artificial Sequence

<220>
<223> Primer

<400> 16
ttattcctca tcctcatttt cattctcttg 30

<210> 17
<211> 26
<212> DNA
<213> Artificial Sequence

<220>
<223> Primer

<400> 17
ctctcttgtg gtgaagatgg aactgt 26

<210> 18
<211> 21
<212> DNA
<213> Artificial Sequence

<220>
<223> Primer

<400> 18
aacgggcaac cattccagta g 21

<210> 19
<211> 21
<212> DNA

<213> Artificial Sequence

<220>
<223> Primer

<400> 19
cactcattac aaagttagca c                                                21

<210> 20
<211> 27
<212> DNA
<213> Artificial Sequence

<220>
<223> Primer

<400> 20
tcagatattt caactcttcc tacggtc                                          27

<210> 21
<211> 25
<212> DNA
<213> Artificial Sequence

<220>
<223> Primer

<400> 21
ccaaaccagg gacaggtgaa ccagt                                            25

<210> 22
<211> 20
<212> DNA
<213> Artificial Sequence

<220>
<223> Primer

<400> 22
gccactgggc cttcagctca                                                  20

## Description

### RELATED APPLICATIONS

This application is a continuation-in-part of and claims priority to U.S. Application Serial No. 09/781,693, filed February 12, 2001 and U.S. Provisional Application Serial No. 60/262,312, filed January 17, 2001, the contents of which are incorporated herein by reference.

### BACKGROUND

A variety of genes that are overexpressed in tumor cells relative to healthy cells have been identified. It is expected that the identification of such genes will provide drug targets for anti-cancer drug development and for cancer diagnostics. The number of steroid receptors (e.g., androgen receptors) in liver tumor cells appears to be increased relative to their adjacent healthy liver cells.

Steroid hormones generally exert their physiological effects by binding to their specific nuclear receptors to form complexes that in turn act as transcription factors. The complexes bind to specific nucleotide sequences (steroid responsive elements) in the promoters of steroid-responsive genes to facilitate transcription of those genes.

### SUMMARY

The invention is based on the discovery of a human protein that is overexpressed in hepatoma cells (relative to adjacent normal cells), and binds to an androgen receptor and augments the ability of the receptor to transactivate an androgen-responsive gene. Thus, this newly discovered human protein is named "androgen receptor complex-associated protein" or "ARCAP." The full-length human ARCAP cDNA (designated SEQ ID NO: 1), with the start and stop codons underlined, is shown below:

CCGGCTCAGGCAGAGCCATGTCTCGGGGTGGCTCCTACCCACACCTGTTGTGGACGTGAGGAAAAGGTCCCTC
GGGCTGGAGGACCCGTCCCGGCTGCGGAGTCGCTACCTGGAAGAAGAGAATTTATCCAAAGATTAAAACTTGA
AGCAACCCTTAATGTGCATGATGGTTGTGTTAATACAATCTGTTGGAATGACACTGGAGAATATATTTTATCTG
GCTCAGATGACACCAAATTAGTAATTAGTAATCCTTACAGCAGAAAGGTTTTGACAACAATTCGTTCAGGGCAC
CGAGCAAACATATTTAGTGCAAAGTTCTTACCTTGTACAAATGATAAACAGATTGTATCCTGCTCTGGAGATGG
AGTAATATTTTATACCAACGTTGAGCAAGATGCAGAAACCAACAGACAATGCCAATTTACGTGTCATTATGGAA
CTACTTATGAGATTATGACTGTACCCAATGACCCTTACACTTTTCTCTCTTGTGGTGAAGATGGAACTGTTAGG
TGGTTTGATACACGCATCAAAACTAGCTGCACAAAAGAAGATTGTAAAGATGATATTTTAATTAACTGTCGACG
TGCTGCCACGTCTGTTGCTATTTGCCCACCAATACCATATTACCTTGCTGTTGGTTGTTCTGACAGCTCAGTAC
GAATATATGATCGGCGAATGCTGGGCACAAGAGCTACAGGGAATTATGCAGGTCGAGGGACTACTGGAATGGTT

GCCCGTTTTATTCCTTCCCATCTTAATAATAAGTCCTGCAGAGTGACATCTCTGTGTTACAGTGAAGATGGTCA
AGAGATTCTCGTTAGTTACTCTTCAGATTACATATATCTTTTTGACCCGAAAGATGATACAGCACGAGAACTTA
AAACTCCTTCTGCGGAAGAGAGAAGAGAAGAGTTGCGACAACCACCAGTTAAGCGTTTGAGACTTCGTGGTGAT
TGGTCAGATACTGGACCCAGAGCAAGGCCGGAGAGTGAACGAGAACGAGATGGAGAGCAGAGTCCCAATGTGTC
ATTGATGCAGAGAATGTCTGATATGTTATCAAGATGGTTTGAAGAAGCAAGTGAGGTTGCACAAAGCAATAGAG
GACGAGGAAGATCTCGACCCAGAGGTGGAACAAGTCAATCAGATATTTCAACTCTTCCTACGGTCCCATCAAGT
CCTGATTTGGAAGTGAGTGAAACTGCAATGGAAGTAGATACTCCAGCTGAACAATTTCTTCAGCCTTCTACATC
CTCTACAATGTCAGCTCAGGCTCATTCGACATCATCTCCCACAGAAAGCCCTCATTCTACTCCTTTGCTATCTT
CTCCAGACAGTGAACAAAGGCAGTCTGTTGAGGCATCTGGACACCACACACATCATCAGTCTGATAACAATAAT
GAAAAGCTGAGCCCCAAACCAGGGACAGGTGAACCAGTTTTAAGTTTGCACTACAGCACAGAAGGAACAACTAC
AAGCACAATAAAACTGAACTTTACAGATGAATGGAGCAGTATAGCATCAAGTTCTAGAGGAATTGGGAGCCATT
GCAAATCTGAGGGTCAGGAGGAATCTTTCGTCCCACAGAGCTCAGTGCAACCACCAGAAGGAGACAGTGAAACA
AAAGCTCCTGAAGAATCATCAGAGGATGTGACAAAATATCAGGAAGGAGTATCTGCAGAAAACCCAGTTGAGAA
CCATATCAATATAACACAATCAGATAAGTTCACAGCCAAGCCATTGGATTCCAACTCAGGAGAAAGAAATGACC
TCAATCTTGATCGCTCTTGTGGGGTTCCAGAAGAATCTGCTTCATCTGAAAAAGCCAAGGAACCAGAAACTTCA
GATCAGACTAGCACTGAGAGTGCTACCAATGAAAATAACACCAATCCTGAGCCTCAGTTCCAAACAGAAGCCAC
TGGGCCTTCAGCTCATGAAGAAACATCCACCAGGGACTCTGCTCTTCAGGACACAGATGACAGTGATGATGACC
CAGTCCTGATCCCAGGTGCAAGGTATCGAGCAGGACCTGGTGATAGACGCTCTGCTGTTGCCCGTATTCAGGAG
TTCTTCAGACGGAGAAAAGAAAGGAAAGAAATGGAAGAATTGGATACTTTGAACATTAGAAGGCCGCTAGTAAA
AATGGTTTATAAAGGCCATCGCAACTCCAGGACAATGATAAAAGAAGCCAATTTCTGGGGTGCTAACTTTGTAA
TGAGTGGTTCTGACTGTGGCCACATTTTCATCTGGGATCGGCACACTGCTGAGCATTTGATGCTTCTGGAAGCT
GATAATCATGTGGTAAACTGCCTGCAGCCACATCCGTTTGACCCAATTTTAGCCTCATCTGGCATAGATTATGA
CATAAAGATCTGGTCACCATTAGAAGAGTCAAGGATTTTTAACCGAAAACTTGCTGATGAAGTTATAACTCGAA
ACGAACTCATGCTGGAAGAAACTAGAAACACCATTACAGTTCCAGCCTCTTTCATGTTGAGGATGTTGGCTTCA
CTTAATCATATCCGAGCTGACCGGTTGGAGGGTGACAGATCAGAAGGCTCTGGTCAAGAGAATGAAAATGAGGA
TGAGGAATAATAAACTCTTTTTGGCAAGCACTTAAATGTTCTGAAATTTGTATAAGACATTTATTATATTTTTT
TCTTTACAGAGCTTTAGTGCAATTTTAAGGTTATGGTTTTTGGAGTTTTTCCCTTTTTTTGGGATAACCTAACA
TTGGTTTGGAATGATTGTGTGCATGAATTTGGGAGATTGTATAAAACAAAACTAGCAGAATGTTTTTAAAACTT
TTTGCCGTGTATGAGGAGTGCTAGAAAATGCAAAGTGCAATATTTTCCCTAACCTTCAAATGTGGAGCTTGGA
TCAATGTTGAAGAATAATTTTCATCATAGTGAAAATGTTGGTTCAAATAAATTTCTACACTTGCCATTTGCATG
TTTGTTGCTTTCTAATTAAAGAAACTGGTTGTTTTAAAAAAAAAAAAAAAGGAATTC (SEQ ID NO:1)

The invention features an isolated nucleic acid having a strand that hybridizes under stringent conditions to a single stranded probe, the sequence of which is SEQ ID NO: 1 or the complement of SEQ ID NO:1. Such a nucleic acid can be at least 15 (e.g., at least 30, 50, 100, 200, 500, or 1000) nucleotides in length.

Hybridization under "stringent conditions" is meant hybridization at 65°C, 0.5 X SSC, followed by washing at 45°C, 0.1 X SSC.

An "isolated nucleic acid" is a nucleic acid the structure of which is not identical to that of any naturally occurring nucleic acid or to that of any fragment of a naturally occurring genomic nucleic acid spanning more than three separate genes. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. Specifically excluded from this definition are nucleic acids present in mixtures of different (i) DNA molecules, (ii) transfected cells, or (iii) cell clones, e.g., as these occur in a DNA library such as a cDNA or genomic DNA library.

The nucleic acids of the invention can be used to diagnose liver cancer by determining whether ARCAP mRNA is being expressed or overexpressed in a tissue or cell. The nucleic acids can be used as primers in PCR-based detection methods, or as labeled probes in nucleic acid blots (e.g., Northern blots).

The following are some examples of PCR primer pairs selected from the human ARCAP cDNA sequence:

(1)	ATGTCTCGGGGTGGCTCCTACCCA (SEQ ID NO:2), and
ACAGTTCCATCTTCACCACAAGAGAG (SEQ ID NO:10);

(2)	ATGTCTCGGGGTGGCTCCTACCC (SEQ ID NO:2), and
CCGATCATATATTCGTACTGAGCTGT (SEQ ID NO:11);

(3)	ATGTCTCGGGGTGGCTCCTACCC (SEQ ID NO:2), and
GACCGTAGGAAGAGTTGAAATATCTGA (SEQ ID NO:12);

(4)	ATGTCTCGGGGTGGCTCCTACCC (SEQ ID NO:2), and
ACTGGTTCACCTGTCCCTGGTTTGG (SEQ ID NO:13);

(5) ATGTCTCGGGTGGCTCCTACCC (SEQ ID NO:2), and
GAGGTCATTTCTTTCTCCTGAGTTGGA (SEQ ID NO:14);

(6)	ATGTCTCGGGGTGGCTCCTACCC (SEQ ID NO:2), and
TGAGCTGAAGGCCCAGTGGC (SEQ ID NO:15);

(7)	ATGTCTCGGGGTGGCTCCTACCC (SEQ ID NO:2), and
TTATTCCTCATCCTCATTTTCATTCTCTTG (SEQ ID NO:16);

(8)	ATGACTGTACCCAATGACCCTTACAC (SEQ ID NO:3), and
CCGATCATATATTCGTACTGAGCTGT (SEQ ID NO:11);

(9)	ATGACTGTACCCAATGACCCTTACAC (SEQ ID NO:3), and
GACCGTAGGAAGAGTTGAAATATACTGA (SEQ ID NO:12);

(10) ATGACTGTACCCAATGACCCTTACAC (SEQ ID NO:3), and
ACTGGTTCACCTGTCCCTGGTTTGG (SEQ ID NO:13);

(11)	ATGACTGTACCCAATGACCCTTACAC (SEQ ID NO:3), and
GAGGTCATTTCTTTCTCCTGAGTTGGA (SEQ ID NO:14);

(12)	ATGACTGTACCCAATGACCCTTACAC (SEQ ID NO:3), and
TGAGCTGAAGGCCCAGTGGC (SEQ ID NO:15);

(13)	ATGACTGTACCCAATGACCCTTACAC (SEQ ID NO:3), and
TTATTCCTCATCCTCATTTTCATTCTCTTG (SEQ ID NO:16);

(14) ACAGCTCAGTACGAATATATGATCGG (SEQ ID NO:4), and
GACCGTAGGAAGAGTTGAAATATACTGA (SEQ ID NO:12);

(15)	ACAGCTCAGTACGAATATATGATCGG (SEQ ID NO:4), and
ACTGGTTCACCTGTCCCTGGTTTGG (SEQ ID NO:13);

(16)	ACAGCTCAGTACGAATATATGATCGG (SEQ ID NO:4), and
TGAGCTGAAGGCCCAGTGGC (SEQ ID NO:15);

(17) ACAGCTCAGTACGAATATATGATCGG (SEQ ID NO:4), and
TTATTCCTCATCCTCATTTTCATTCTCTTG (SEQ ID NO:16);

(18)	CTACTGGAATGGTTGCCGTT (SEQ ID NO:5), and
GACCGTAGGAAGAGTTGAAATATACTGA (SEQ ID NO:12);

(19)	CTACTGGAATGGTTGCCGTT (SEQ ID NO:5), and
ACTGGTTCACCTGTCCCTGGTTTGG (SEQ ID NO:13);

(20)	CTACTGGAATGGTTGCCGTT (SEQ ID NO:5), and
TGAGCTGAAGGCCCAGTGGC (SEQ ID NO:15);

(21)	CTACTGGAATGGTTGCCGTT (SEQ ID NO:5), and
TTATTCCTCATCCTCATTTTCATTCTCTTG (SEQ ID NO:16);

(22) ATGCAGAGAATGTCTGATATGTTATC (SEQ ID NO:6), and
GACCGTAGGAAGAGTTGAAATATACTGA (SEQ ID NO:12);

(23)	ATGCAGAGAATGTCTGATATGTTATC (SEQ ID NO:6), and
ACTGGTTCACCTGTCCCTGGTTTGG (SEQ ID NO:13);

(24)	ATGCAGAGAATGTCTGATATGTTATC (SEQ ID NO:6), and
TGAGCTGAAGGCCCAGTGGC (SEQ ID NO:15);

(25)	ATGCAGAGAATGTCTGATATGTTATC (SEQ ID NO:6), and
TTATTCCTCATCCTCATTTTCATTCTCTTG (SEQ ID NO:16);

(26)	TCCAACTCAGGAGAAAGAAATGACCTC (SEQ ID NO:7), and
TGAGCTGAAGGCCCAGTGGC (SEQ ID NO:15);

(27)	TCCAACTCAGGAGAAAGAAATGACCTC (SEQ ID NO:7), and
TTATTCCTCATCCTCATTTTCATTCTCTTG (SEQ ID NO:16);

(28)	GCCACTGGGCCTTCAGCTCA (SEQ ID NO:8), and
TTATTCCTCATCCTCATTTTCATTCTCTTG (SEQ ID NO:16);

(29)	GTGCTAACTTTGTAATGAGTG (SEQ ID NO:9), and
TTATTCCTCATCCTCATTTTCATTCTCTTG (SEQ ID NO:16);

(30) CTCTCTTGTGGTGAAGATGGAACTGT (SEQ ID NO:17), and
CCGATCATATATTCGTACTGAGCTGT (SEQ ID NO:11);

(31) CTCTCTTGTGGTGAAGATGGAACTGT (SEQ ID NO:17), and
AACGGGCAACCATTCCAGTAG (SEQ ID NO:18);

(32)	CTCTCTTGTGGTGAAGATGGAACTGT (SEQ ID NO:17), and
GACCGTAGGAAGAGTTGAAATATCTGA (SEQ ID NO:12);

(33)	CTCTCTTGTGGTGAAGATGGAACTGT (SEQ ID NO:17), and
ACTGGTTCACCTGTCCCTGGTTTGG (SEQ ID NO:13);

(34)	CTCTCTTGTGGTGAAGATGGAACTGT (SEQ ID NO:17), and
GAGGTCATTTCTTTCTCCTGAGTTGGA (SEQ ID NO:14);

(35) CTCTCTTGTGGTGAAGATGGAACTGT (SEQ ID NO:17), and
TGAGCTGAAGGCCCAGTGGC (SEQ ID NO:15);

(36)	CTCTCTTGTGGTGAAGATGGAACTGT (SEQ ID NO:17), and
CACTCATTACAAAGTTAGCAC (SEQ ID NO:19);

(37) CTCTCTTGTGGTGAAGATGGAACTGT (SEQ ID NO:17), and
TTATTCCTCATCCTCATTTTCATTCTCTTG (SEQ ID NO:16);

(38) TCAGATATTTCAACTCTTCCTACGGTC (SEQ ID NO:20), and
ACTGGTTCACCTGTCCCTGGTTTGG (SEQ ID NO:13);

(39)	TCAGATATTTCAACTCTTCCTACGGTC (SEQ ID NO:20), and
GAGGTCATTTCTTTCTCCTGAGTTGGA (SEQ ID NO:14);

(40)	TCAGATATTTCAACTCTTCCTACGGTC (SEQ ID NO:20), and
TGAGCTGAAGGCCCAGTGGC (SEQ ID NO:15);

(41)	TCAGATATTTCAACTCTTCCTACGGTC (SEQ ID NO:20), and
CACTCATTACAAAGTTAGCAC (SEQ ID NO:19);

(42)	TCAGATATTTCAACTCTTCCTACGGTC (SEQ ID NO:20), and
TTATTCCTCATCCTCATTTTCATTCTCTTG (SEQ ID NO:16);

(43)	CCAAACCAGGGACAGGTGAACCAGT (SEQ ID NO:21), and
GAGGTCATTTCTTTCTCCTGAGTTGGA (SEQ ID NO:14);

(44) CCAAACCAGGGACAGGTGAACCAGT (SEQ ID NO:21), and
TGAGCTGAAGGCCCAGTGGC (SEQ ID NO:15);

(45) CCAAACCAGGGACAGGTGAACCAGT (SEQ ID NO:21), and
CACTCATTACAAAGTTAGCAC (SEQ ID NO:19);

(46) CCAAACCAGGGACAGGTGAACCAGT (SEQ ID NO:21), and
TTATTCCTCATCCTCATTTTCATTCTCTTG (SEQ ID NO:16);

(47)	GCCACTGGGCCTTCAGCTCA (SEQ ID NO:22), and
CACTCATTACAAAGTTAGCAC (SEQ ID NO:19).

Also within the scope of this invention is a pair of amplification primers consisting of a first primer and a second primer, each primer being 20-40 (e.g., 20-35 and 20-30) nucleotides in length. The first primer contains SEQ ID NO:2 and the second primer contains SEQ ID NO:10, 11, 12, 13, 14, 15, or 16; the first primer contains SEQ ID NO:3 and the second primer contains SEQ ID NO: 11, 12, 13, 14, 15, or 16; the first primer contains SEQ ID NO:4 and the second primer contains SEQ ID NO: 12, 13, 15, or 16; the first primer contains SEQ ID NO:5 and the second primer contains SEQ ID NO: 12, 13, 15, or 16; the first primer contains SEQ ID NO:6 and the second primer contains SEQ ID NO: 12, 13, 15, or 16; the first primer contains SEQ ID NO:7 and the second primer contains SEQ ID NO: 15 or 16; the first primer contains SEQ ID NO:8 and the second primer contains SEQ ID NO: 16; the first primer contains SEQ ID NO:9 and the second primer contains SEQ ID NO: 16; the first primer contains SEQ ID NO: 17 and the second primer contains SEQ ID NO:11, 18, 12, 13, 14, 15, 16, or 19; the first primer contains SEQ ID NO:20 and the second primer contains SEQ ID NO:13, 14, 15, 16, or 19; the first primer contains SEQ ID NO:21 and the second primer contains SEQ ID NO:14, 15, 16, or 19; or the first primer contains SEQ ID NO:22 and the second primer contains SEQ ID NO: 19.

A nucleic acid obtained from amplification of a human nucleic acid template with one of the above-described primer pairs can be used as a hybridization probe for detection of human ARCAP mRNA.

Further within the scope of this invention is a kit for diagnosing liver cancer. The kit contains one or more of the primers or probes described above. It can include other components such as a DNA polymerase, a PCR buffer, or a solid support on which one or more of the above-described primers or probes are localized or immobilized.

The present invention provides a method of detecting liver cancer. The method involves providing a sample (e.g., a blood sample such as a buffy coat sample) from a subject and determining the ARCAP gene expression level, e.g., by amplification and hybridization with one of the above-described primer pairs and probes, respectively. If the ARCAP gene expression level in the sample is higher than that in a sample prepared from a normal subject, it indicates that the subject has liver cancer. The subject can be a person at high risk of developing liver cancer, e.g., a hepatitis B, hepatitis C, or liver cirrhosis patient, or his or her relative, or a liver cancer patient's relative. Unexpectedly, liver cancer is diagnosed using this method at an accuracy of above 98%, which is far more better than the 70% accuracy of the commonly used method based on α-fetoprotein (AFP) levels.

The present invention also provides a method of staging liver cancer. The method involves providing a sample (e.g., a blood sample such as a buffy coat sample) from a liver cancer patient and determining the ARCAP gene expression level, e.g., by amplification and hybridization with one of the above-described primer pairs and probes, respectively. The cancer stage of the patient is determined by comparing the ARCAP gene expression level in the sample with those in samples prepared from other patients having liver cancer of various stages.

Other features or advantages of the present invention will be apparent from the following detailed description, and also from the claims.

### DETAILED DESCRIPTION

This invention relates to a newly identified ARCAP gene that is overexpressed in hepatocellular carcinoma cells relative to normal liver cells. In addition to differential expression, ARCAP was found to bind to and augment the transactivation activity of an androgen receptor. These observations suggest that ARCAP activates, via an androgen receptor complex, mitogenic genes that are androgen-responsive (i.e., genes whose promoters contain androgen responsive elements), that overexpression of ARCAP leads to cancer by facilitating androgen receptor-mediated transactivation of androgen-responsive mitogenic genes, and that inhibition of ARCAP expression or activity would reduce expression of these androgen-responsive mitogenic genes and revert cancer cells to a more normal phenotype. Consequently, ARCAP is a new cancer drug target.

The ARCAP molecules are useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the ARCAP molecules may be detected, and may be correlated with one or more biological states *in vivo.* For example, the ARCAP molecules may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a liver tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include those described in Koomen *et al.* (2000) *J. Mass. Spectrom.* 35: 258-264; and James (1994) *AIDS Treatment News Archive* 209.

The ARCAP molecules are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug *in vivo.* Pharmacodynamic markers are of particular use in increasing the sensitivity of detection of drug effects, particularly when the drug is administered in low doses. Since even a small amount of a drug may be sufficient to activate multiple rounds of marker (e.g., an ARCAP marker) transcription or expression, the amplified marker may be in a quantity which is more readily detectable than the drug itself. Also, the marker may be more easily detected due to the nature of the marker itself; for example, using the methods described herein, anti-ARCAP antibodies may be employed in an immune-based detection system for an ARCAP protein marker, or ARCAP-specific radiolabeled probes may be used to detect an ARCAP mRNA marker. Furthermore, the use of a pharmacodynamic marker may offer mechanism-based prediction of risk due to drug treatment beyond the range of possible direct observations. Examples of the use of pharmacodynamic markers in the art are described in Matsuda *et al.* US 6,033,862; Hattis *et al.* (1991) Env. Health Perspect. 90: 229-238; Schentag (1999) *Am. J. Health-Syst. Pharm.* 56 Suppl. 3: S21-S24; and Nicolau (1999) *Am, J. Health-Syst. Pharm.* 56 Suppl. 3: S16-S20.

The ARCAP molecules are also useful as pharmacogenomic markers. As used herein, a "pharmacogenomic marker" is an objective biochemical marker which correlates with a specific clinical drug response or susceptibility in a subject (see, e.g., McLeod *et al.* (1999) *Eur. J. Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (e.g., ARCAP protein or RNA) for specific tumor markers in a subject, a drug or course of treatment may be selected which is optimized for the treatment of the specific tumor likely to be present in the subject. Similarly, the presence or absence of a specific sequence mutation in ARCAP DNA may correlate with ARCAP drug response. The use of pharmacogenomic markers therefore permits the application of the most appropriate treatment for each subject without having to administer the therapy.

The expression level of the ARCAP gene can be determined by amplification with a pair of primers, e.g., SEQ ID NO:2 and SEQ ID NO:10, 11, 12, 13, 14, 15, or 16; SEQ ID NO:3 and SEQ ID NO: 11, 12, 13, 14, 15, or 16; SEQ ID NO:4 and SEQ ID NO:12, 13, 15, or 16; SEQ ID NO:5 and SEQ ID NO: 12, 13, 15, or 16; SEQ ID NO:6 and SEQ ID NO: 12, 13, 15, or 16; SEQ ID NO:7 and SEQ ID NO: 15 or 16; SEQ ID NO:8 and SEQ ID NO: 16; SEQ ID NO:9 and SEQ ID NO: 16; SEQ ID NO: 17 and SEQ ID NO: 11, 18, 12, 13, 14, 15, 16, or 19; SEQ ID NO:20 and SEQ ID NO:13, 14, 15, 16, or 19; SEQ ID NO:21 and SEQ ID NO: 14, 15, 16, or 19; or SEQ ID NO:22 and SEQ ID NO:19. Alternatively, it can be determined by hybridization (e.g., Northern blot) with a nucleic acid probe, e.g., a nucleic acid obtained from amplification of a human nucleic acid template with one of the just-described primer pairs.

The amplification template can be DNA (i.e., cDNA) or RNA (i.e., total RNA or mRNA), in a purified or unpurified form. It can be obtained from a biological sample (e.g., a biopsy such as a liver biopsy, or a blood sample such as a buffy coat sample) from a patient.

Typically, a primer is 14-40 nucleotides in length (PCR Application Manual, Boehringer Mannheim, 1995, page 37). Also within the scope of this invention are primers that are a few nucleotides shorter than the above examples (i.e., SEQ ID Nos:2-22) but still can be used to detect human ARCAP mRNA. Such primers can be identified by determining whether they can be used to produce a PCR product of an expected size from a human ARCAP nucleic acid template. Primers longer (e.g., 20-40, 20-35, or 20-30 nucleotides in length) than the above examples can also be used to detect human ARCAP mRNA. For instance, additional nucleotides can be added to either the 5'-end or the 3'-end according to the human ARCAP gene sequence. Non-human ARCAP gene sequences can be added to the 5'-end. An example of a non-human ARCAP sequence is a sequence containing a restriction site used to facilitate cloning of the amplification product.

The amplification product can be visualized by resolving it on a gel (e.g., an agarose or polyacrylamide gel) through electrophoresis, or by hybridizing it to a probe. The probes can be immobilized on the surface of a solid support, such as a membrane (a nylon-membrane or a nitrocellulose membrane), a glass, or a plastic polymer. Immobilization of probes to a membrane can be achieved by baking at 80°C or UV cross-linking. The probes can also be covalently linked to a material (e.g., poly-lysine) coated on the surface of a glass. Alternatively, the probes can be synthesized de novo at precise positions on a solid substrate. See Schena et al., 1995, Science 270: 467; Kozal et al., 1996, Nature Medicine 2(7): 753; Cheng et al., 1996, Nucleic Acids Res. 24(2): 380; Lipshutz et al., 1995, BioTechniques 19(3): 442; Pease et al., 1994, Proc. Natl. Acad. Sci. USA 91: 5022; Fodor et al., 1993, Nature 364: 555; and Fodor et al., WO 92/10092. To facilitate the detection, a labeled amplification product can be generated with a labeled amplification primer. Alternatively, the labeling can be done, chemically or enzymatically, after amplification. Examples of labeling reagents include, but are not limited to, a fluorescent molecule (e.g., fluorescein and rhodamine), a radioactive isotope (e.g., ³²P and ¹²⁵I), a colorimetric reagent, and a chemiluminescent reagent. Biotin and digoxgenin are frequently used for colorimetric detection on a membrane or a plastic polymer. Fluorescent labels, such as Cy3 and Cy5, are widely used for detection on a glass. In addition, artificial tagging tails (e.g., a protein or its antibody) can be conjugated to the 5'-end of the primers or either end of the probes. See Stetsenko and Gait, 2000, J. Org. Chem. 65(16): 4900.

The specific example below is to be construed as merely illustrative, and not limitative of the remainder of the disclosure in any way whatsoever. Without further elaboration, it is believed that one skilled in the art can, based on the description herein, utilize the present invention to its fullest extent. All publications recited herein-are hereby incorporated by reference in their entirety.

### Isolation of Buffy Coat from Blood Cells

1. Withdraw 10 ml whole blood per sample.
2. Isolate blood cells by centrifugation at 20°C, 3,500 rpm (PK-131R, ALC International S.r.1., Italy) for 15 min.
3. Remove white blood cells (WBC) with a scaled-dropper, and then transfer cells to a 15-mL centrifuge tube.
4. Add 3 volumes of Phosphate Buffered Saline (PBS) into white blood cells (WBC:PBS = 1:3 ), and mix them evenly.
5. Add 4 mL mixed WBC slowly to a 15-mL centrifuge tube filled with 2 mL Ficoll-Paque ( Ficoll-paque:Sample = 1:2 ). Do not disturb the interface between the WBC and Ficoll-Paque, and make sure that the WBC is layered on top of Ficoll-Paque.
6. Centrifuge at 20°C, 3,500 rpm (PK-131R, ALC International S.r.1., Italy) for 15min.
7. Remove the second layer of white cells with a scaled dropper (2 mL) and transfer cells to a 15-mL centrifuge tube.
8. Add 2 mL PBS into white blood cells (WBC:PBS = 1:1), and mix them evenly.
9. Centrifuge at 20°C, 3,500 rpm (PK-131R, ALC International S.r.1., Italy) for 15 min.
10. Discard the supernatant.
11. Resuspand the white pellet with 1 mL PBS.
12. Centrifuge at 20°C, 12,000 rpm (PK-131R, ALC International S.r.1., Italy) for 20 min.
13. Discard the supernatant, and keep the pellet (denoted as Buffy coat) in two separated eppendorfs. The Buffy coat can be saved at -20°C, if necessary.

### Isolation of Total RNA

1. Add 1 mL TRIzol Reagent to 1.5-mL eppendorf with previously prepared buffy coat.
2. Pipet the buffy coat several times to resuspend the cells.
3. Break the cells by passing through 18g needle 20 times.
4. Add 200 µL chloroform to cell mixture (1/5 volume of TRIzol Reagent), and vortex them vigorously.
5. Keep the cell mixture on ice (4°C) for 10 min.
6. Centrifuge at 4°C, 12,000 rpm (PK-131R, ALC International S.r.1., Italy) for 10 min.
7. Transfer the upper layer of the solution (approximately about 600 µL of RNA solution) to a 1.5-mL eppendorf.
8. Add equal volume of isopropanol (around 600 µL) to RNA solution, and mix them evenly.
9. Keep the mixture on the ice (4°C) for 30 min.
10. Centrifuge at 4 °C, 12,000 rpm (PK-131R, ALC International S.r.1., Italy) for 30 min.
11. Remove the supernatant and save the RNA pellete.
12. Add 200 µL 75% Alcl-DEPC (Diethyl Pyrocabonate, Sigma) to the RNA pellet.
13. Centrifuge at 4 °C, 12,000 rpm (PK-131R, ALC International S.r.1., Italy) for 10 min.
14. Repeat step 11 to remove the supernatant and save the RNA pellete.
15. Add 10 µL of ddH₂O-DEPC to resuspend the RNA.
16. Keep the RNA solution at 4°C overnight.

75% alcohol-DEPC: 75 mL absolute alcohol mixed with 25 mL ddH₂O-DEPC to make 75% Alc-DEPC, kept at 4 °C.

ddH₂O-DEPC: 1 mL DEPC mixed with 1,000 mL dd H₂O to make ddH₂O-DEPC, kept at 4°C after autoclaving.

### RT-PCR

1. Mix 1 µL prepared RNA with 99 µL ddH₂O, determine the concentration of RNA using RNA/DNA Calculator (GeneQuant Pro Classic; Amersham Biosciences). For each RT-PCR, 10 µg of total RNA is needed.
2. Mix RNA with H₂O-DEPC to make a final volume of 17 µL.
3. Denature RNA at 65 °C for 10 min.
4. Prepare a reverse transcription mixture:

| | |
|---|---|
| 5 × First Buffer | 6λ |
| dNTPs | 2λ |
| 0.1 M DTT | 2λ |
| Oligo(dT) (1 µg/µL) | 1λ |
| RNase inhibitor (27.5 u/µL) | 1λ |
| M-MLV reverse transcriptase (200 u/µL) | 1λ |
| Denatured total RNA (10 µg) | 17λ |

8. Incubate at 37°C for 60 min followed by 65°C for 10 min to obtain cDNA (can be stored at -20°C).
9. Prepare a PCR mixture:

| | |
|---|---|
| cDNA | 1λ |
| Forward primer | 0.1λ |
| Reverse primer | 0.1λ |
| dNTPs | 0.2λ |
| Taq DNA polymerase (5 u/µL) | 0.5λ |
| 10×PCR Buffer | 2.5λ |
| ddH₂O | 20.6λ |

10. Detect PCR products by electrophoresis in a 1.0% agarose gel at 110V for 20 min.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. A pair of amplification primers consisting of a first primer and a second primer, wherein
the first primer contains SEQ ID NO:2 and the second primer contains SEQ ID NO:10, 11, 12, 13, 14, 15, or 16;
the first primer contains SEQ ID NO:3 and the second primer contains SEQ ID NO:11, 12, 13, 14, 15, or 16;
the first primer contains SEQ ID NO:4 and the second primer contains SEQ ID NO:12, 13, 15, or 16;
the first primer contains SEQ ID NO:5 and the second primer contains SEQ ID NO:12, 13, 15, or 16;
the first primer contains SEQ ID NO:6 and the second primer contains SEQ ID NO:12, 13, 15, or 16;
the first primer contains SEQ ID NO:7 and the second primer contains SEQ IDNO:15 or 16;
the first primer contains SEQ ID NO: 8 and the second primer contains SEQ ID NO:16;
the first primer contains SEQ ID NO:9 and the second primer contains SEQ ID NO: 16;
the first primer contains SEQ ID NO: 17 and the second primer contains SEQ ID NO:11, 18, 12, 13, 14, 15, 16, or 19;
the first primer contains SEQ ID NO:20 and the second primer contains SEQ ID NO:13, 14, 15, 16, or 19;
the first primer contains SEQ ID NO:21 and the second primer contains SEQ ID NO:14, 15, 16, or 19; or
the first primer contains SEQ ID NO:22 and the second primer contains SEQ ID NO: 19;
each primer being 20-40 nucleotides in length.

2. The pair of primers of claim 1, wherein each primer is 20-35 nucleotides in length.

3. The pair of primers of claim 2, wherein each primer is 20-30 nucleotides in length.

4. The pair of primers of claim 1, wherein
the first primer is SEQ ID NO:2 and the second primer is SEQ ID NO: 10, 11, 12,13,14,15, or 16;
the first primer is SEQ ID NO:3 and the second primer is SEQ ID NO:11, 12, 13,14,15, or 16;
the first primer is SEQ ID NO:4 and the second primer is SEQ ID NO: 12, 13, 15, or 16;
the first primer is SEQ ID NO:5 and the second primer is SEQ ID NO:12, 13, 15, or 16;
the first primer is SEQ ID NO:6 and the second primer is SEQ ID NO:12, 13, 15, or 16;
the first primer is SEQ ID NO:7 and the second primer is SEQ ID NO: 15 or 16;
the first primer is SEQ ID NO:8 and the second primer is SEQ ID NO: 16;
the first primer is SEQ ID NO:9 and the second primer is SEQ ID NO: 16,
the first primer is SEQ ID NO: 17 and the second primer is SEQ ID NO: 11, 18, 12, 13, 14, 15, 16, or 19;
the first primer is SEQ ID NO:20 and the second primer is SEQ ID NO:13, 14, 15, 16, or 19;
the first primer is SEQ ID NO:21 and the second primer is SEQ ID NO: 14, 15, 16, or 19; or
the first primer is SEQ ID NO:22 and the second primer is SEQ ID NO:19.

5. A nucleic acid obtained from amplification of a human nucleic acid template with a pair of primers of claim 1.

6. The nucleic acid of claim 5, wherein each primer is 20-35 nucleotides in length.

7. The nucleic acid of claim 6, wherein each primer is 20-30 nucleotides in length.

8. A method of detecting liver cancer, the method comprising:
providing a sample from a subject, and
determining a gene expression level of an androgen receptor complex-associated protein by amplification with a pair of primers of claim 1 or by hybridization with a nucleic acid of claim 5,
wherein the gene expression level in the sample, if higher than that in a sample prepared from a normal subject, indicates that the subject has liver cancer.

9. The method of claim 8, wherein the sample is a blood sample.

10. The method of claim 9, wherein the sample is a buffy coat sample.

11. The method of claim 8, wherein the subject is a hepatitis B patient or a relative thereof.

12. The method of claim 11, wherein the sample is a blood sample.

13. The method of claim 12, wherein the sample is a buffy coat sample.

14. The method of claim 8, wherein the subject is a hepatitis C patient or a relative thereof.

15. The method of claim 14, wherein the sample is a blood sample.

16. The method of claim 15, wherein the sample is a buffy coat sample.

17. The method of claim 8, wherein the subject is a liver cirrhosis patient or a relative thereof.

18. The method of claim 17, wherein the sample is a blood sample.

19. The method of claim 18, wherein the sample is a buffy coat sample.

20. The method of claim 8, wherein the subject is a relative of a liver cancer patient.

21. The method of claim 20, wherein the sample is a blood sample.

22. The method of claim 21, wherein the sample is a buffy coat sample.

23. A method of staging liver cancer, the method comprising:
providing a sample from a liver cancer patient, and
determining a gene expression level of an androgen receptor complex-associated protein by amplification with a pair of primers of claim 1 or by hybridization with a nucleic acid of claim 5,
wherein the gene expression level in the sample, upon comparison with those in samples prepared from other patients having liver cancer of various stages, indicates a cancer stage of the patient.

24. The method of claim 23, wherein the sample is a blood sample.

25. The method of claim 24, wherein the sample is a buffy coat sample.
